(19) Europäisches Patentamt / European Patent Office / Office européen des brevets

(11) **EP 4 111 965 B1**

(12) **EUROPEAN PATENT SPECIFICATION**

(45) Date of publication and mention
of the grant of the patent:
**06.05.2026 Bulletin 2026/19**

(21) Application number: **22181849.5**

(22) Date of filing: **29.06.2022**

(51) International Patent Classification (IPC):
**A61B 5/145** (2006.01)

(52) Cooperative Patent Classification (CPC):
**A61B 5/14532; A61B 5/1495; A61B 5/7264**

(54) **GLUCOSE SENSOR IDENTIFICATION USING ELECTRICAL PARAMETERS**

GLUKOSESENSOR-IDENTIFIZIERUNG UNTER VERWENDUNG VON ELEKTRISCHEN PARAMETERN

IDENTIFICATION D'UN CAPTEUR DE GLUCOSE À L'AIDE DE PARAMÈTRES ÉLECTRIQUES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **01.07.2021 US 202117365803**

(43) Date of publication of application:
**04.01.2023 Bulletin 2023/01**

(73) Proprietor: **Medtronic MiniMed, Inc.**
**Northridge, CA 91325-1219 (US)**

(72) Inventors:
• **GEE, Elaine**
**Northridge, 91325 (US)**
• **PICCININI, Francesca**
**Northridge, 91325 (US)**
• **ZHOU, Li**
**Northridge, 91325 (US)**
• **TRAN, Chi A**
**Northridge, 91325 (US)**
• **BATMANGHELICH, Farhad**
**Northridge, 91325 (US)**
• **NAVA-GUERRA, Leonardo**
**Northridge, 91325 (US)**

• **ARGUELLES MORALES, Juan Enrique**
**Northridge, 91325 (US)**
• **PATEL, Anuj M.**
**Northridge, 91325 (US)**
• **AROYAN, Sarkis D.**
**Northridge, 91325 (US)**

(74) Representative: **Zimmermann & Partner**
**Patentanwälte mbB et al**
**P.O. Box 330 920**
**80069 München (DE)**

(56) References cited:
**EP-A1- 3 712 728        US-A1- 2004 223 155**
**US-A1- 2012 262 298**

• **EREN GULTEPE ET AL: "Improving clustering performance using independent component analysis and unsupervised feature learning", HUMAN-CENTRIC COMPUTING AND INFORMATION SCIENCES, BIOMED CENTRAL LTD, LONDON, UK, vol. 8, no. 1, 23 August 2018 (2018-08-23), pages 1 - 19, XP021259956, DOI: 10.1186/S13673-018-0148-3**

**Description**

TECHNICAL FIELD

This disclosure relates to glucose sensors.

BACKGROUND

Glucose sensors are configured to detect and/or quantify the amount of glucose in a patient's body (e.g., interstitial glucose or possibly blood glucose), which enables patients and medical personnel to monitor physiological conditions within the patient's body. In some examples, it may be beneficial to monitor glucose levels on a continuing basis (e.g., in a diabetic patient). Thus, glucose sensors have been developed for use in obtaining an indication of glucose levels in a diabetic patient. Such indications are useful in monitoring and/or adjusting a treatment regimen, which typically includes administration of insulin to the patient.

A patient can measure their blood glucose (BG) using a BG measurement device (i.e., glucose meter), such as a test strip meter. A continuous glucose measurement system (or a continuous glucose monitor (CGM)) may be configured to determine interstitial glucose; although possible for CGM to determine blood glucose. A hospital hemacue may also be used to determine glucose level CGMs may be beneficial for patients who desire to take more frequent glucose measurements. Some example CGM systems include subcutaneous (or short-term) sensors and implantable (or long-term) sensors. A CGM system may execute an initialization sequence when the CGM is inserted into a patient. The initialization sequence may speed up sensor equilibration and may allow a CGM system to provide reliable glucose measurements earlier.

US 2012/0262298 A1 relates to an advanced analyte sensor calibration and error detection.

US 2004/0223155 A1 relates to a method of characterizing spectrometer instruments and providing calibration models to compensate for instrument variation.

SUMMARY

In general, this disclosure describes techniques for calibrating glucose sensors (e.g., a continuous glucose monitor or "CGM") based on one or more electrical parameters. More particularly, this disclosure describes techniques and devices for determining electrical parameters of a glucose sensor in vitro and/or in vivo and identifying a cluster for the glucose sensor that may be used for configuring the glucose sensor.

A glucose sensor may comprise configuration information related to manufacturing the glucose sensor, which may be used to determine a glucose level of a patient. To set the configuration information, a process may comprise measuring the glucose level of a patient with the glucose sensor in vivo and measuring the glucose level of a patient with a pre-configured device. In this example, the configuration information may be set (e.g., by a clinician) based on a comparison of the glucose level measured by the glucose sensor and the glucose level measured by the pre-configured device.

In some examples, the glucose level measured by the glucose sensor may be an interstitial glucose level, and the glucose level measured by the pre-configured device may be blood glucose level. In such examples, a processor may be configured to convert the interstitial glucose level to a blood glucose level (e.g., such as by scaling and offsetting), and compare the blood glucose level determined from the interstitial glucose level to the blood glucose level measured by the pre-configured device.

In accordance with the techniques of the disclosure, configuration information of a glucose monitor may be set based on one or more electrical parameters measured in vitro. Electrical parameters include a voltage, an electrical current (e.g., iSig), or an impedance. In general, the electrical current (iSig) flowing through the sensing (e.g., working) electrode of a glucose sensor is indicative of the glucose level in the patient's interstitial fluid. For example, processing circuitry may measure the one or more electrical parameters after the glucose monitor is manufactured and before the glucose monitor is implanted into a patient.

The processing circuitry identifies a cluster for the glucose sensor based on the one or more electrical parameters. Each cluster of the plurality of clusters identifies configuration information. The configuration information may include a correction factor for improving an accuracy of the glucose sensor. The glucose sensor may be calibrated with configuration information (e.g., the correction factor) based on a cluster identified for the glucose sensor. For example, in response to identifying a particular cluster for the glucose device, the processing circuitry may calibrate the glucose sensor with a respective set of configuration information that are assigned to the particular cluster. In this way, a glucose sensor may be calibrated without relying on in vivo calibration techniques, which may improve an accuracy of the glucose device. Moreover, glucose sensors that are assigned to a cluster associated with inaccurate glucose sensors may be pruned, which may help to improve an accuracy and/or reliability of a resulting set of glucose monitors. A more optimal configuration of the glucose sensor may also improve the longevity of the glucose sensor.

In one example, a method for calibrating a glucose sensor includes determining, by one or more processors, a set of electrical parameters for the glucose sensor , wherein the electrical parameters comprise a voltage at the glucose sensor, an electrical current for the glucose sensor, or an impedance for the glucose sensor, and determining, by the one or more processors, a cluster for the glucose sensor from a plurality of clusters based on the set of electrical parameters. Each cluster of the plurality of clusters identifies respective configuration information. The method further includes configuring, by the one or more processors, the glucose sensor to determine a glucose level of a patient based on configuration information identified by the determined cluster.

In another example, a device for calibrating a glucose sensor, the device includes a memory and one or more processors implemented in circuitry and in communication with the memory. The one or more processors are configured to determine a set of electrical parameters for the glucose sensor, wherein the electrical parameters comprise a voltage at the glucose sensor, an electrical current for the glucose sensor, or an impedance for the glucose sensor, and determine a cluster for the glucose sensor from a plurality of clusters based on the set of electrical parameters. Each cluster of the plurality of clusters identifies respective configuration information. The one or more processors are further configured to configure the glucose sensor to determine a glucose level of a patient based on configuration information identified by the determined cluster.

In one example, a non-transitory computer-readable storage medium having stored thereon instructions that, when executed, configures a processor to determine a set of electrical parameters for a glucose sensor wherein the electrical parameters comprise a voltage at the glucose sensor, an electrical current for the glucose sensor, or an impedance for the glucose sensor, and determine a cluster for the glucose sensor from a plurality of clusters based on the set of electrical parameters. Each cluster of the plurality of clusters identifies respective configuration information. The instructions further configures the processor to configure the glucose sensor to determine a glucose level of a patient based on configuration information identified by the determined cluster.

The details of one or more examples are set forth in the accompanying drawings and the description below. Other features, objects, and advantages will be apparent from the description and drawings, and from the claims.

BRIEF DESCRIPTION OF DRAWINGS

FIG. 1 is a block diagram illustrating an example glucose level management system in accordance with one or more examples described in this disclosure.
FIG. 2 is a block diagram illustrating an example glucose sensor in accordance with one or more examples described in this disclosure.
FIG. 3 is a block diagram illustrating example sensor electrodes and a voltage being applied to the sensor electrodes according to an example of the disclosure.
FIG. 4 is a block diagram illustrating example processing circuitry configured to determine a cluster for a glucose sensor in accordance with one or more examples described in this disclosure.
FIG. 5 is a conceptual diagram illustrating example of determining initial in vitro and in vivo features for determining a cluster for a glucose sensor in accordance with one or more examples described in this disclosure.
FIG. 6 is a conceptual diagram illustrating an example of determining a cluster for a glucose sensor in accordance with one or more examples described in this disclosure.
FIG. 7 is a conceptual diagram illustrating an example process of applying a principal component analysis (PCA) algorithm in accordance with one or more examples described in this disclosure.
FIG. 8 is a conceptual diagram illustrating an example applying a reconstruction independent component analysis (RICA) algorithm in accordance with one or more examples described in this disclosure.
FIG. 9 is a conceptual diagram illustrating an example of model validation.
FIG. 10 is a flowchart illustrating an example technique of the disclosure.
FIG. 11 is a flowchart illustrating another example technique of the disclosure.

DETAILED DESCRIPTION

In general, this disclosure describes techniques for calibrating glucose sensors (e.g., a continuous glucose monitor or "CGM") based on one or more electrical parameters. More particularly, this disclosure describes techniques and devices for identifying electrical parameters of a glucose sensor in vitro and identifying a cluster for the glucose sensor. In some examples, the glucose sensor may use the identified cluster to configure with configuration information associated with the identified cluster.

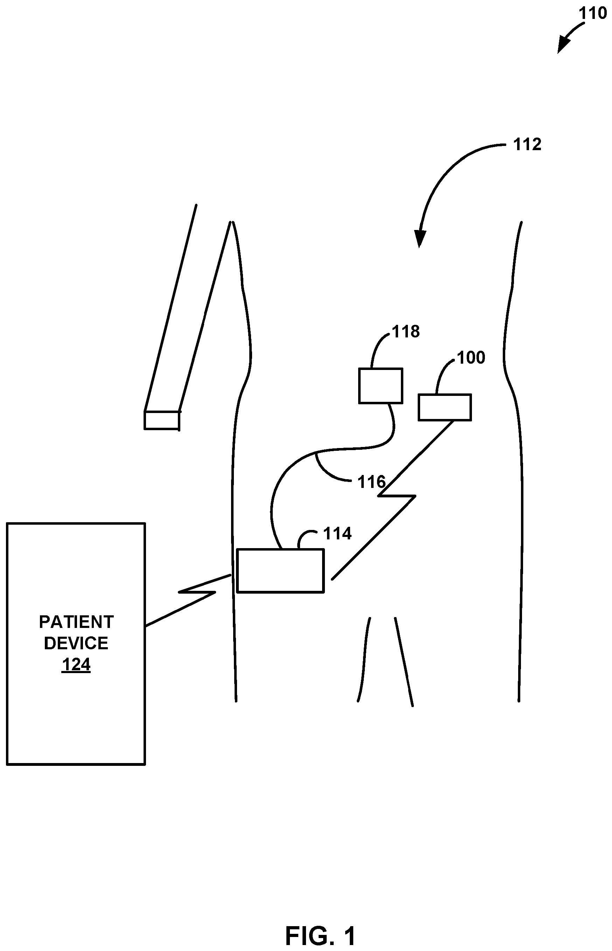

FIG. 1 is a block diagram illustrating an example glucose level management system in accordance with one or more examples described in this disclosure. FIG. 1 illustrates system 110 that includes insulin pump 114, tubing 116, infusion set 118, monitoring device 100 (e.g., a glucose level monitoring device comprising a glucose sensor), and patient device 124. Insulin pump 114 may be described as a tethered pump, because tubing 116 tethers insulin pump 114 to infusion set 18. In some examples, rather than utilizing a tethered pump system comprising insulin pump 114, tubing 116, infusion set 118, and/or monitoring device 100, patient 112 may utilize a patch pump. Instead of delivering insulin via tubing and an infusion set, a pump patch may deliver insulin via a cannula extending directly from an insulin pump. In some examples, a glucose sensor may also be integrated into such an insulin pump (e.g., a so-called "all-in-one (AIO) insulin pump").

Patient 112 may be diabetic (e.g., Type 1 diabetic or Type 2 diabetic), and therefore, the glucose level in patient 112 may be controlled with delivery of supplemental insulin. For example, patient 112 may not produce sufficient insulin to control the glucose level or the amount of insulin that patient 112 produces may not be sufficient due to insulin resistance that patient 112 may have developed.

To receive the supplemental insulin, patient 112 may carry insulin pump 114 that couples to tubing 116 for delivery of insulin into patient 112. Infusion set 118 may connect to the skin of patient 112 and include a cannula to deliver insulin into patient 112. Monitoring device 100 may also be coupled to patient 112 to measure glucose level in patient 112. Insulin pump 114, tubing 116, infusion set 118, and monitoring device 100 may together form an insulin pump system. One example of the insulin pump system is the MINIMED™ 670G insulin pump system by MEDTRONIC MINIMED, INC. However, other examples of insulin pump systems may be used and the example techniques should not be considered limited to the MINIMED™ 670G insulin pump system. For example, the techniques described in this disclosure may be utilized with any insulin pump and/or glucose monitoring system that includes an in vivo glucose sensor (e.g., a continuous glucose monitor or other in vivo glucose sensor).

Monitoring device 100 may include a sensor that is inserted under the skin of patient 112 (e.g., in vivo), such as near the stomach of patient 112 or in the arm of patient 112 (e.g., subcutaneous connection). The sensor of monitoring device 100 may be configured to measure the interstitial glucose level, which is the glucose found in the fluid between the cells of patient 112. Monitoring device 100 may be configured to continuously or periodically sample the glucose level and rate of change of the glucose level over time.

In one or more examples, insulin pump 114, monitoring device 100, and/or the various components illustrated in FIG. 1, may together form a closed-loop therapy delivery system. For example, patient 112 may set a target glucose level, usually measured in units of milligrams per deciliter, on insulin pump 114. Insulin pump 114 may receive the current glucose level from monitoring device 100 and, in response, may increase or decrease the amount of insulin delivered to patient 112. For example, if the current glucose level is higher than the target glucose level, insulin pump 114 may increase the insulin. If the current glucose level is lower than the target glucose level, insulin pump 114 may temporarily cease delivery of the insulin. Insulin pump 114 may be considered as an example of an automated insulin delivery (AID) device. Other examples of AID devices may be possible, and the techniques described in this disclosure may be applicable to other AID devices.

Insulin pump 114 and monitoring device 100 may be configured to operate together to mimic some of the ways in which a healthy pancreas works. Insulin pump 114 may be configured to deliver basal dosages, which are small amounts of insulin released continuously throughout the day. There may be times when glucose levels increase, such as due to eating or some other activity that patient 112 undertakes. Insulin pump 114 may be configured to deliver bolus dosages on demand in association with food intake or to correct an undesirably high glucose level in the bloodstream. In one or more examples, if the glucose level rises above a target level, then insulin pump 114 may deliver a bolus dosage to address the increase in glucose level. Insulin pump 114 may be configured to compute basal and bolus dosages and deliver the basal and bolus dosages accordingly. For instance, insulin pump 114 may determine the amount of a basal dosage to deliver continuously and then determine the amount of a bolus dosage to deliver to reduce glucose level in response to an increase in glucose level due to eating or some other event.

Accordingly, in some examples, monitoring device 100 may sample glucose levels for determining rate of change in glucose level over time. Monitoring device 100 may output the glucose level to insulin pump 114 (e.g., through a wireless link connection like Bluetooth). Insulin pump 114 may compare the glucose level to a target glucose level (e.g., as set by patient 112 or a clinician) and adjust the insulin dosage based on the comparison. In some examples, insulin pump 114 may adjust insulin delivery based on a predicted glucose level (e.g., where glucose level is expected to be in the next 30 minutes).

As described above, patient 112 or a clinician may set one or more target glucose levels on insulin pump 114. There may be various ways in which patient 112 or the clinician may set a target glucose level on insulin pump 114. As one example, patient 112 or the clinician may utilize patient device 124 to communicate with insulin pump 114. Examples of patient device 124 include mobile devices, such as smartphones, tablet computers, laptop computers, and the like. In some examples, patient device 124 may be a special programmer or controller (e.g., a dedicated remote control device) for insulin pump 114. Although FIG. 1 illustrates one patient device 124, in some examples, there may be a plurality of patient devices. For instance, system 110 may include a mobile device and a dedicated wireless controller, each of which is an example of patient device 124. For ease of description only, the example techniques are described with respect to patient device 124 with the understanding that patient device 124 may be one or more patient devices.

Patient device 124 may also be configured to interface with monitoring device 100. As one example, patient device 124 may receive information from monitoring device 100 through insulin pump 114, where insulin pump 114 relays the information between patient device 124 and monitoring device 100. As another example, patient device 124 may receive information (e.g., glucose level or rate of change of glucose level) directly from monitoring device 100 (e.g., through a wireless link).

In one or more examples, patient device 124 may comprise a user interface with which patient 112 or the clinician may control insulin pump 114. For example, patient device 124 may comprise a touchscreen that allows patient 112 or the clinician to enter a target glucose level. Additionally or alternatively, patient device 124 may comprise a display device that outputs the current and/or past glucose level. In some examples, patient device 124 may output notifications to patient 112, such as notifications if the glucose level is too high or too low, as well as notifications regarding any action that patient 112 needs to take.

Monitoring device 100 may comprise configuration information (e.g., one or more correction factors) related to manufacturing the glucose sensor, which may be used to determine a glucose level of patient 112. In accordance with the techniques of the disclosure, configuration information of monitoring device 100 may be set based on one or more electrical parameters measured in vitro. As used herein, in vitro may refer to when sensor(s) of monitoring device 100 are positioned outside of a human subject. In contrast, in vivo may refer to when one or more sensors of monitoring device 100 are at least partially positioned inside of a human patient.

Electrical parameters include a voltage, an electrical current (e.g., iSig), or an impedance. In general, the electrical current (iSig) flowing through the sensing (e.g., working) electrode of a glucose sensor is indicative of the blood glucose level in the patient's interstitial fluid.

For example, in response to receiving an indication of a particular cluster for monitoring device 100, monitoring device 100 calibrate with a respective set of configuration information that are assigned to the particular cluster. Each cluster identifies respective configuration information. For example, glucose sensors assigned to a cluster may comprise one or more common manufacturing features (e.g., defects, manufacturing tolerance ranges, etc.) that would benefit from a common set of configuration information (e.g., correction factors) to mitigate or even eliminate inaccuracies in determining a glucose level resulting from the one or more manufacturing features. Techniques described herein may use electrical parameters that may be determined in vitro to determine a cluster for a glucose sensor. In this way, monitoring device 100 may be calibrated without relying on in vivo calibration techniques, which may improve an accuracy of the glucose device. A more optimal configuration of monitoring device 100 may also improve the longevity of monitoring device 100.

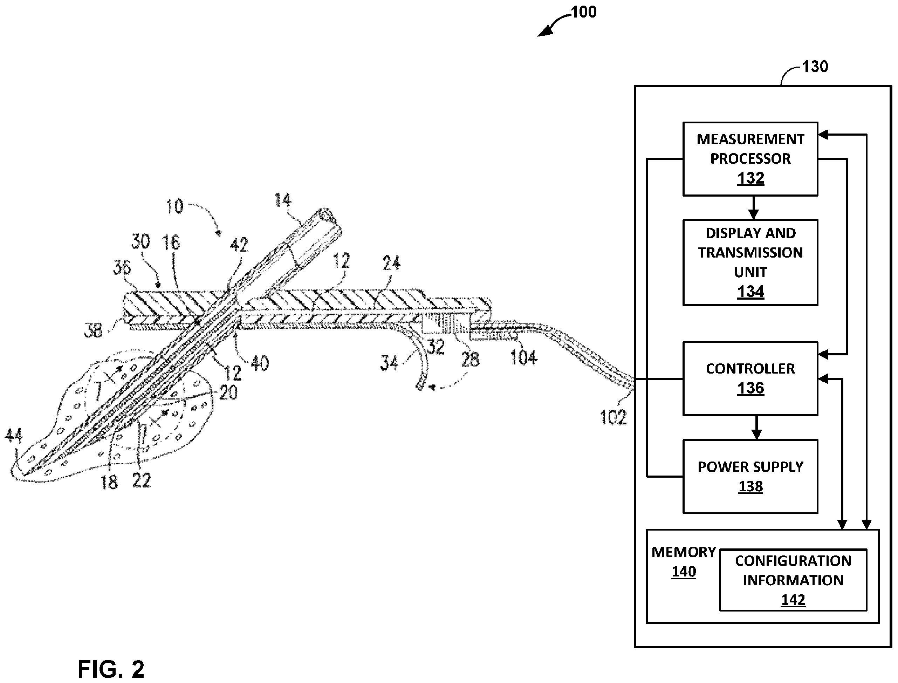

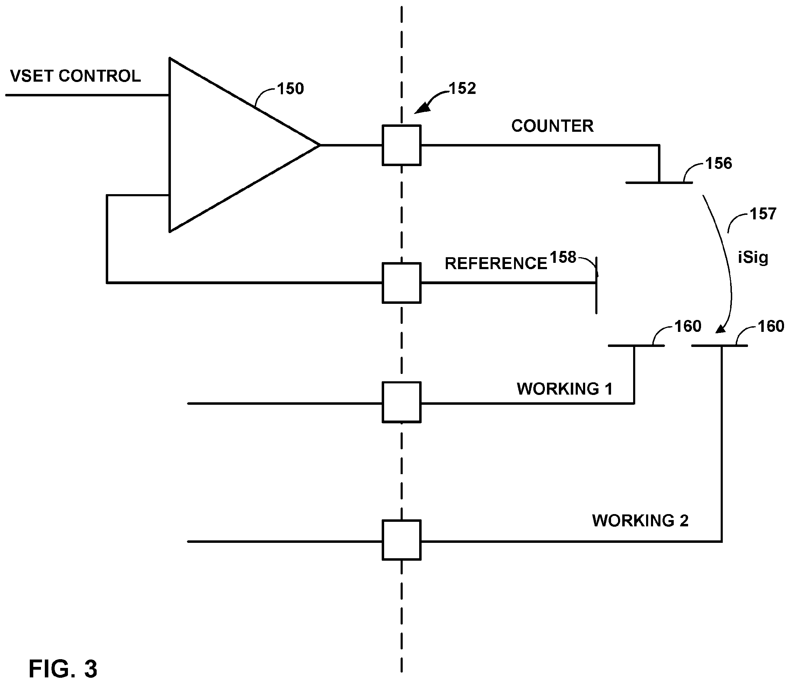

FIG. 2 is a block diagram illustrating monitoring device 100 in more detail. In particular, FIG. 2 is a perspective view of a subcutaneous sensor insertion set and a block diagram of sensor electronics device 130 of monitoring device 100 according to an example of the disclosure. As illustrated in FIG. 2, subcutaneous sensor set 10 is provided for subcutaneous placement of an active portion of flexible glucose sensor 12 at a selected site in the body of patient 112. The subcutaneous or percutaneous portion of sensor set 10 includes a hollow, slotted insertion needle 14, and cannula 16. Needle 14 is used to facilitate quick and easy subcutaneous placement of cannula 16 at the subcutaneous insertion site. Inside cannula 16 is glucose sensing portion 18 of glucose sensor 12, which is configured to expose one or more glucose sensor electrodes 20 to the bodily fluids (e.g., blood or interstitial fluid) of patient 112 through window 22 formed in cannula 16. In one example, one or more glucose sensor electrodes 20 may include a counter electrode, a reference electrode, and one or more working electrodes. Examples of the counter electrode, reference electrode, and working electrode(s) are described in more detail with respect to FIG. 3. After insertion, insertion needle 14 is withdrawn to leave cannula 16 with glucose sensing portion 18 and glucose sensor electrodes 20 in place at the selected insertion site.

In particular examples, subcutaneous sensor set 10 facilitates accurate placement of flexible thin film electrochemical glucose sensor 12 of the type used for monitoring specific blood parameters representative of a condition of patient 112. Glucose sensor 12 monitors glucose levels in the body, and may be used in conjunction with automated or semi-automated medication infusion pumps of the external or implantable type as described above to control delivery of insulin to patient 112.

Particular examples of flexible electrochemical glucose sensor 12 are constructed in accordance with thin film mask techniques to include elongated thin film conductors embedded or encased between layers of a selected insulative material such as polyimide film or sheet, and membranes. Glucose sensor electrodes 20 at a tip end of glucose sensing portion 18 are exposed through one of the insulative layers for direct contact with patient blood or other body fluids, when glucose sensing portion 18 (or active portion) of glucose sensor 12 is subcutaneously placed at an insertion site. Glucose sensing portion 18 is joined to connection portion 24 that terminates in conductive contact pads, or the like, which are also exposed through one of the insulative layers. In other examples, other types of implantable sensors, such as chemical based, optical based, or the like, may be used.

Connection portion 24 and the contact pads are generally adapted for a direct wired electrical connection to a suitable monitor or sensor electronics device 130 for monitoring a condition of patient 112 in response to signals derived from glucose sensor electrodes 20. Connection portion 24 may be conveniently connected electrically to the monitor or sensor electronics device 130 or by connector block 28. Thus, in accordance with examples of the disclosure, subcutaneous sensor sets 10 may be configured or formed to work with either a wired or a wireless characteristic monitor system.

Glucose sensor electrodes 20 may be used in a variety of sensing applications and may be configured in a variety of ways. For example, glucose sensor electrodes 20 may be used in physiological parameter sensing applications in which some type of biomolecule is used as a catalytic agent. For example, glucose sensor electrodes 20 may be used in a glucose and oxygen sensor having a glucose oxidase (GOx) enzyme catalyzing a reaction with glucose sensor electrodes 20. Glucose sensor electrodes 20, along with a biomolecule or some other catalytic agent, may be placed in a human body in a vascular or non-vascular environment. For example, glucose sensor electrodes 20 and biomolecules may be placed in a vein and be subjected to a blood stream, or may be placed in a subcutaneous or peritoneal region of the human body.

Sensor electronics device 130 may include measurement processor 132, display and transmission unit 134, controller 136, power supply 138, and memory 140. Sensor electronics device 130 may be coupled to the sensor set 10 by cable 102 through a connector that is electrically coupled to connector block 28 of connection portion 24. In other examples, the cable may be omitted and sensor electronics device 130 may include an appropriate connector for direct connection to connection portion 104 of sensor set 10. Sensor set 10 may be modified to have connector portion 104 positioned at a different location, e.g., on top of the sensor set to facilitate placement of sensor electronics device 130 over the sensor set.

In examples of the disclosure, measurement processor 132, display and transmission unit 134, and controller 136 may be formed as separate semiconductor chips. However, other examples may combine measurement processor 132, display and transmission unit 134, and controller 136 into a single or multiple customized semiconductor chips. In general, measurement processor 132 may be configured to receive a current and/or voltage from glucose sensor electrodes sensors 20. Glucose sensor electrodes sensors 20 may generate a sensor signal indicative of a concentration of a physiological characteristic being measured. For example, the sensor signal may be indicative of a glucose reading. The sensor signal may be measured at a working electrode of glucose sensor electrodes sensors 20. In an example of the disclosure, the sensor signal may be a current (e.g., iSig) measured at the working electrode. In another example of the disclosure, the sensor signal may be a voltage (e.g., Vcounter) measured at the working electrode of glucose sensor electrodes sensors 20. Electrical parameters, such as voltage, electrical current, and impedance, may be measured in vitro and may be referred to herein as "in vitro features."

An example of an impedance parameter may include electrochemical impedance spectroscopy (EIS). EIS may provide additional information in the form of sensor impedance and impedance-related parameters at different frequencies. Moreover, for certain ranges of frequencies, impedance and/or impedance-related data are substantially glucose independent. Such glucose independence enables the use of a variety of EIS-based markers or indicators for not only producing a robust, highly-reliable sensor glucose value (through fusion methodologies), but also assessing the condition, health, age, and efficiency of individual electrode(s) and of the overall sensor substantially independently of the glucose-dependent Isig.

For example, analysis of the glucose-independent impedance data provides information on the efficiency of a sensor of monitoring device 100 with respect to how quickly it hydrates and is ready for data acquisition using, e.g., values for 1 kHz real-impedance, 1 kHz imaginary impedance, and Nyquist Slope (to be described in more detail hereinbelow). Moreover, glucose-independent impedance data provides information on potential occlusion(s) that may exist on the sensor membrane surface, which occlusion(s) may temporarily block passage of glucose into the sensor and thus cause the signal to dip (using, e.g., values for 1 kHz real impedance). In addition, glucose-independent impedance data provides information on loss of sensor sensitivity during extended wear-potentially due to local oxygen deficit at the insertion site-using, e.g., values for phase angle and/or imaginary impedance at 1 kHz and higher frequencies.

Within the context of electrode redundancy and EIS, a fusion algorithm may be used to take the diagnostic information provided by EIS for each redundant electrode (e.g., in systems with multiple working electrodes) and assess the reliability of each electrode independently. Weights, which are a measure of reliability, may then be added for each independent signal, and a single fused signal may be calculated that can be used to generate sensor glucose values as seen by the patient/subject.

The combined use of redundancy, sensor diagnostics using EIS, and EIS-based fusion algorithms may allow for an overall CGM system that is more reliable than systems that do not use EIS. Redundancy is advantageous in at least two respects. First, redundancy removes the risk of a single point of failure by providing multiple signals. Second, providing multiple (working) electrodes where a single electrode may be sufficient allows the output of the redundant electrode to be used as a check against the primary electrode, thereby reducing, and perhaps eliminating, the need for frequent calibrations. In addition, EIS diagnostics may scrutinize the health of each electrode autonomously without the need for a reference glucose value (finger stick), thereby reducing the number of reference values required. However, the use of EIS technology and EIS diagnostic methods is not limited to redundant systems, e.g., those having more than one working electrode. Rather, EIS may be advantageously used in connection with single- and/or multiple-electrode sensors of monitoring device 100.

Measurement processor 132 receives the sensor signal (e.g., a measured current or voltage) after the sensor signal is measured at glucose sensor electrodes sensors 20 (e.g., the working electrode). Measurement processor 132 may receive the sensor signal and calibrate the sensor signal utilizing reference values. In an example of the disclosure, the reference values are stored in a reference memory (e.g., memory 140) and provided to measurement processor 132. Based on the sensor signals and the reference values, measurement processor 132 may determine a glucose measurement. Measurement processor 132 store the glucose measurements in memory 140. The sensor measurements may be sent to display and transmission unit 134 to be either displayed on a display in a housing of monitoring device 100 or transmitted to an external device.

Memory 140 may be any type of memory device and may be configured to store glucose measurements produced by measurement processor 132, reference values used to determine glucose measurements from sensor signals, or other data used and/or produced by measurement processor 132 and/or controller 136. In some examples, memory 140 may further store software and/or firmware that is executable by measurement processor 132 and/or controller 136. As will be explained in more detail below, memory 140 may further configuration information 142. Configuration information 142 may include data that may be executed by controller 136 to configure sensor electronics device 130.

Sensor electronics device 130 may be a monitor which includes a display to display physiological characteristics readings. Sensor electronics device 130 may also be installed in a desktop computer, a pager, a television including communications capabilities, a laptop computer, a server, a network computer, a personal digital assistant (PDA), a portable telephone including computer functions, an infusion pump including a display, a glucose sensor including a display, and/or a combination infusion pump/glucose sensor. Sensor electronics device 130 may be housed in a mobile phone, a network device, a home network device, or an appliance connected to a home network.

Power supply 138 may be a battery. The battery can include three series silver oxide 357 battery cells. In other examples, different battery chemistries may be utilized, such as lithium based chemistries, alkaline batteries, nickel metalhydride, or the like, and a different number of batteries may be used. Sensor electronics device 130 provides power to the sensor set 10 via power supply 138 through cable 102 and cable connector 104.

Controller 136 may be a processor, digital signal processors (DSPs), application specific integrated circuits (ASICs), field programmable gate arrays (FPGAs), or any other equivalent integrated or discrete logic circuitry. In some examples controller 136 may be configured to execute software program code and/or firmware that causes power supply 138 to supply a specific voltage or current to glucose sensor electrodes sensors 20. Glucose sensor electrodes sensors 20 may receive the voltage level or value. In an example of the disclosure, a counter electrode of glucose sensor electrodes sensors 20 may receive the reference voltage from power supply 138. The application of the voltage level causes glucose sensor electrodes sensors 20 to create a sensor signal (e.g., a current through a working electrode) indicative of a concentration of a physiological characteristic being measured (e.g., blood glucose).

FIG. 3 is a block diagram illustrating example sensor electrodes and a voltage being applied to the sensor electrodes according to an example of the disclosure. In the example in FIG. 3, an operational amplifier (op amp) 150 or other servo controlled device may connect to glucose sensor electrodes 20 through a circuit/electrode interface 152. Op amp 150, utilizing feedback through glucose sensor electrodes 20, attempts to maintain a prescribed voltage between reference electrode 158 and a working electrode 160 (e.g., VSET) by adjusting the voltage at counter electrode 156. In some examples, the voltage at reference electrode 158 is 850 mv.

Current 157 (iSig) may then flow from a counter electrode 156 to a working electrode 160. Counter electrode 156 balances the chemical reaction that is occurring at working electrode 160. Measurement processor 132 of FIG. 2 may measure current 157 to determine the electrochemical reaction between glucose sensor electrodes 20 and a biomolecule of a sensor that has been placed in the vicinity of the sensor electrodes and used as a catalyzing agent. The circuitry disclosed in FIG. 3 may be utilized in a long-term or implantable sensor or may be utilized in a short-term or subcutaneous sensor.

Returning to FIG. 2, as discussed above, due to manufacturing variances, monitoring device 100 may provide inaccurate readings to patient 112. As such, configuration information may "correct" for manufacturing variances, which will improve an accuracy of measurements of glucose level of patient 112. In accordance with the techniques of this disclosure, controller 136 may cause sensor electronics 130 to configure monitoring device 100 using configuration information 142. In particular, controller 136 may configure with configuration information associated with an identified cluster for sensor electronics device 130. In this way, sensor electronics device 130 may be calibrated without relying on in vivo calibration techniques, which may improve an accuracy of sensor electronics device 130. Moreover, sensor electronics devices that are assigned to a cluster associated with inaccurate glucose sensors may be pruned, which may help to improve an accuracy and/or reliability of a resulting set of sensor electronics devices.

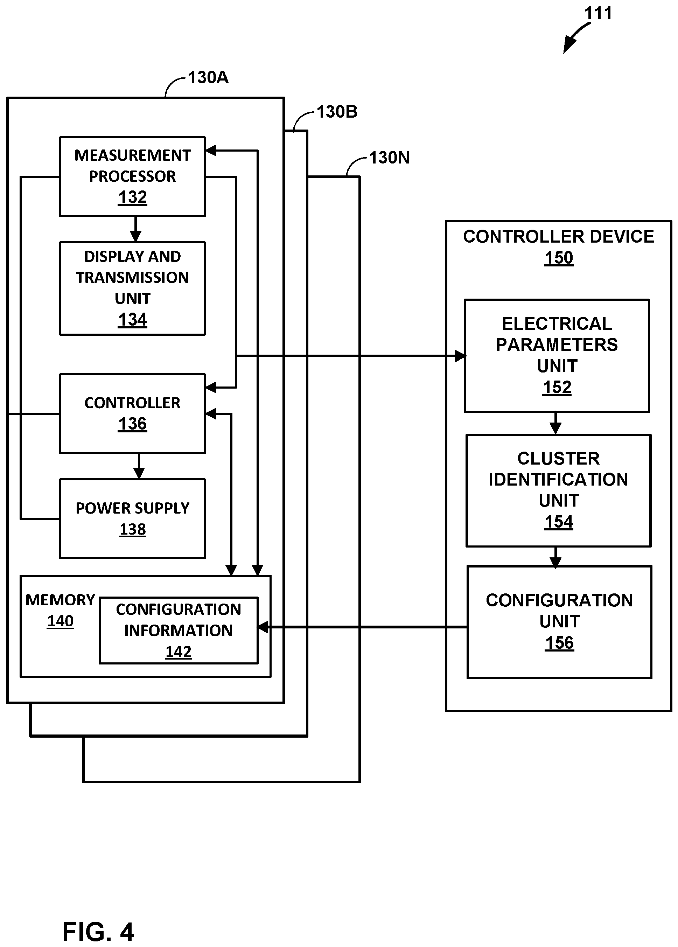

FIG. 4 is a block diagram illustrating example processing circuitry configured to determine a cluster for a glucose sensor. As shown in FIG. 4, measurement processor 132 may provide a sensor signal to controller device 150. In one example, the sensor signal is a current (iSig) through a working electrode of glucose sensor electrodes 20. Sensor signal accumulator 200 of controller 136 may accumulate current values over a period of time. Sensor signal analyzer 202 may then calculate a rate of change of the current values. For example, sensor signal analyzer 202 may calculate the slope of a plot of the current (e.g., iSig).

Electrical parameters unit 152 may determine a set of electrical parameters for glucose sensor 130A of glucose sensors 130A-130N (collectively, glucose sensors 130). Cluster identification unit 154 may determine a cluster for the glucose sensor based on the set of electrical parameters. Each cluster may identify respective configuration information. For example, glucose sensors assigned to a cluster may comprise one or more common manufacturing features (e.g., defects, manufacturing tolerance ranges, etc.) that would benefit from a common set of configuration information (e.g., correction factors) to mitigate or even eliminate inaccuracies in determining a glucose level resulting from the one or more manufacturing features. Techniques described herein may use electrical parameters that may be determined in vitro to determine a cluster for a glucose sensor. Configuration unit 156 may configure glucose sensor 130A to determine a glucose level of a patient based on configuration information 142 associated with the cluster. In this way, controller device 150 may improve a configuration of controller device 150 using configuration information (e.g.., one or more correction factors) that are determined using electrical parameters detected in vitro, which may improve an accuracy of controller device 150.

In other examples of the disclosure, controller 136 may use sensor signals other than current (iSig) through the working electrode to determine adjustments to an initialization sequence. In other examples, controller 136 may accumulate and measure changes in electro chemical impedance spectroscopy (EIS) (real and imaginary impedance at different frequencies), voltage at a counter electrode, and/or current through a background electrode. A background electrode is similar to a working electrode, but does not include Gox layer for breaking down and sensing glucose. Like current through a working electrodes, larger slopes/changes in these other sensor signals may indicate a need for a higher VSET, whole lower slopes/changes in these other sensor signals may indicate a need for a lower Vset at the working electrode.

Development of a new CGM sensor may include measuring identifying qualities (e.g., signal features) that relate to good sensor performance with respect to calibrated sensor glucose outputs (e.g., sensor glucose accuracy, sensor lifetime, and iCGM performance). This disclosure describes techniques for identifying raw signal features measured from the sensor signals (e.g., sensor glucose calibration independent signals) that can separate sensors into different categories with respect to sensor glucose dependent performance. This may allow sensor stratification in a sensor glucose calibration algorithm in an independent manner.

This framework can be used to categorize sensors for predicted sensor glucose performance by organizing through raw sensor signals in the following applications: new CGM development, CGM product maintenance and tracking, and quality control form CGM manufacturing.

Techniques described herein may tie in vitro and in vivo features that derive from sensor signals measured in vitro and in vivo (e.g., clinical data) to good performance with respect to estimating sensor glucose. The in vitro and in vivo features may be algorithm agnostic and may be derived from sensor measurements from in vitro testing (e.g., SITS testing) and/or clinical feasibility studies, respectively. The analysis techniques may be scaled to any CGM platform where measurements are available. The in vitro and in vivo features may be used to evaluate sensor performance in the absence of a finalized sensor glucose algorithm as the techniques may be adaptable to be applied to any sensor glucose calibration algorithm. Techniques described herein may also support the in vitro test limit setup, which can potentially increase the production yield and improve manufacturing control.

For example, controller device 150 may extract CGM signal features from invitro data testing sensor performance under different conditions to create a feature library. Controller device 150 may extract CGM signal signatures from in vivo data to create a feature library. Cluster identification unit 154 of controller device 150 may apply clustering techniques. For example, cluster identification unit 154 may apply unsupervised machine learning techniques, such as clustering analysis, to separate sensors into different groups based on in vivo feature similarity. The partitioning of glucose sensors into different groups may not require a sensor glucose algorithm and can separate the glucose sensors into groups with different sensor performance, such as mean absolute relative difference (MARD), accuracy, etc. Cluster identification unit 154 may create linear regression models with, for example, iSig, Ventr, and EIS features to evaluate sensor glucose performance of sensors clustered into groups.

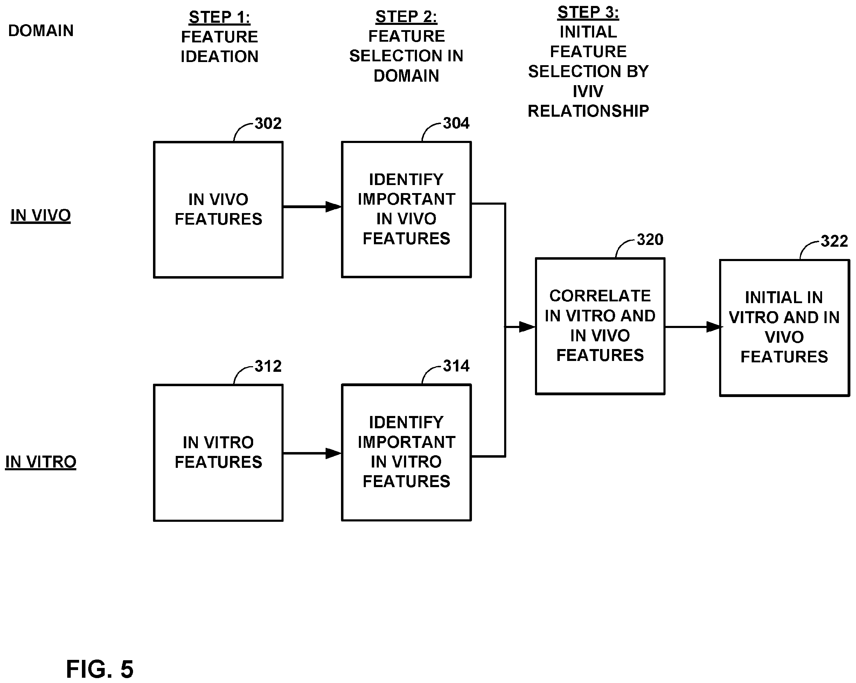

FIG. 5 is a conceptual diagram illustrating example of determining initial in vitro and in vivo features for determining a cluster for a glucose sensor. Controller device 150, for example, electrical parameters unit 152, may determine in vivo features (302) and identify important in vivo features (304). For example, controller device 150 and/or a human user may determine initial in vivo features based on signal modeling and/or experience and controller device 150 may identify important in vivo features related to good sensor performance (e.g., clustering and sensor glucose interim model development). Similarly, controller device 150, for example, electrical parameters unit 152, may determine in vitro features (312) and identify important in vitro features (314). For example, controller device 150 and/or a human user may select in vitro features based on signal modeling and/or experience and controller device 150 may correlate in vitro features to identify independent in vitro features.

Controller device 150, for example, electrical parameters unit 152, may correlate in vitro and in vivo features (320). For example, controller device 150 may correlate higher glucose accuracy with a iSig within a threshold range. Controller device 150 may correlate in vitro and in vivo features based on a linear regression of the invitro features and the in vivo features. In vitro features that are good predictors of the in vivo counterpart can show a high covariance and high correlation coefficient, meaning that variations in the values of the in vitro features can explain the variations observed in the values of the in vivo counterpart. When no linear relationship is expected but instead the correlation is driven by a monotonic relationship, this correlation value can be estimated as a function of the feature ranks (e.g. Spearman correlation). Initial independent in vitro feature selection can be achieved through these techniques, as well as important correlations between in vitro and in vivo can be identified.

Controller device 150, for example, electrical parameters unit 152, may determine initial in vitro and in vivo features (322). For example, controller device 152 may determine that the glucose accuracy is an initial in vivo feature and, in response to the correlation of glucose accuracy the iSig, determine that iSig is an initial in vitro feature.

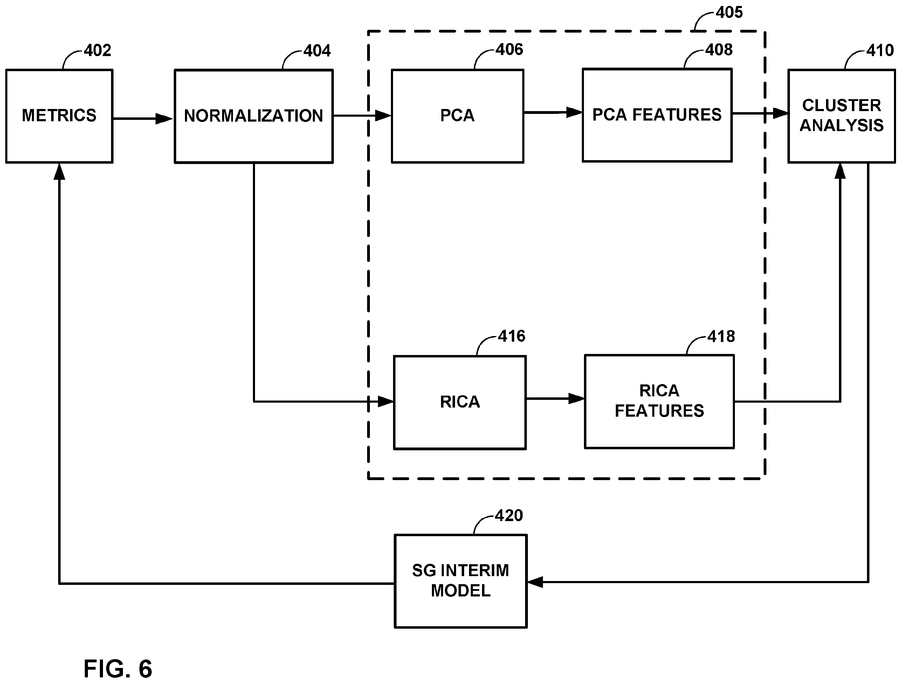

FIG. 6 is a conceptual diagram illustrating an example of determining a cluster for a glucose sensor. In the example of FIG. 6, controller device 150, for example, electrical parameters unit 152, may determine one or more metrics (402). In some examples, metrics may be generated using clinical patient data (e.g., finger stick measurements). Metrics may include calibration "cal" factors, which may refer to a ratio of blood glucose (BG) to sensor signal. Sensor signal may be adjusted by an offset value. For example, the cal ratio may be equal to (BG)/(Isig-offset). For example, controller device 150, for example, electrical parameters unit 152, may perform one or more of steps 302-322 of FIG. 5. In this example, controller device 150, for example, electrical parameters unit 152, may perform normalization of the metrics (404). For instance, controller device 150 may normalize the metrics using one or more of a number of standard deviations from the mean value of BG (Zscore), normalize the range from -1 to 1 (range of -1 1) or an Euclidean norm of the Zscore (Zscore to 2norm).

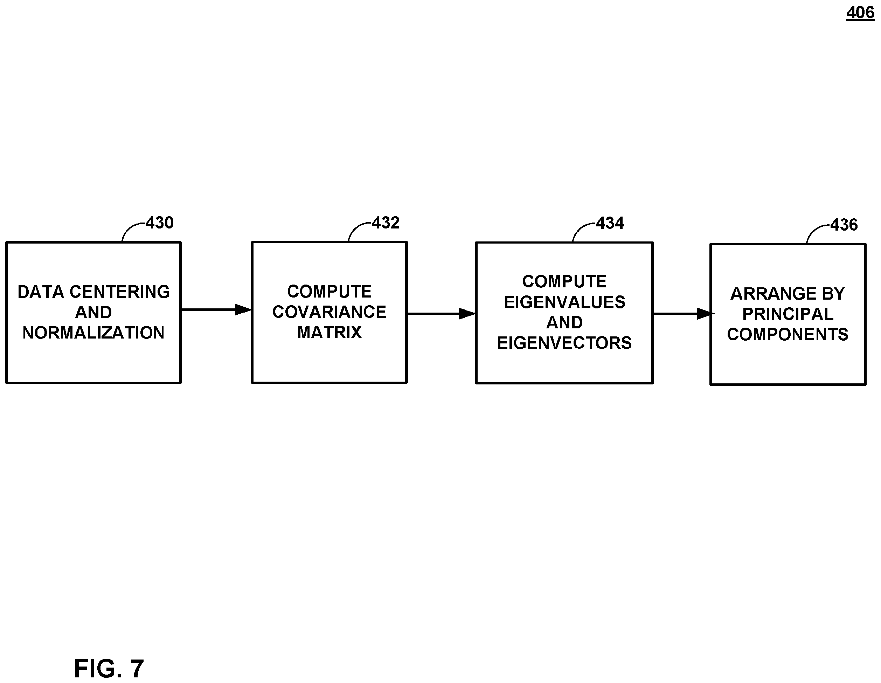

Controller device 150, for example, electrical parameters unit 152, may apply an unsupervised feature learning algorithm (e.g., a machine learning algorithm) to compress dimensionality and retain raw information (405). For example, controller device 150, for example, electrical parameters unit 152, may apply a principal component analysis (PCA) algorithm (406) to generate PCA features 408 for identifying the cluster. An example process of applying a PCA algorithm is shown in FIG. 7. In some examples, the PCA algorithm may use a ranked list of features. For instance, controller device 150 may determine a correlation of in vitro features to in vivo features and select a weight value for in vitro features based on the correlation of the in vitro features to the in vivo features. In some instances, controller device 150 may set a weight value for an in vitro feature to be higher as the correlation of the in vitro feature to the in vivo features increases and may set the weight value for an in vitro feature to be lower as the correlation of the in vitro feature to the in vivo features decreases. PCA may refer to an unsupervised feature learning algorithm to convert original features into low dimensional and statistically independent features. An example of PCA may be found at ufldl.stanford.edu/tutorial/unsupervised/PCAWhitening/.

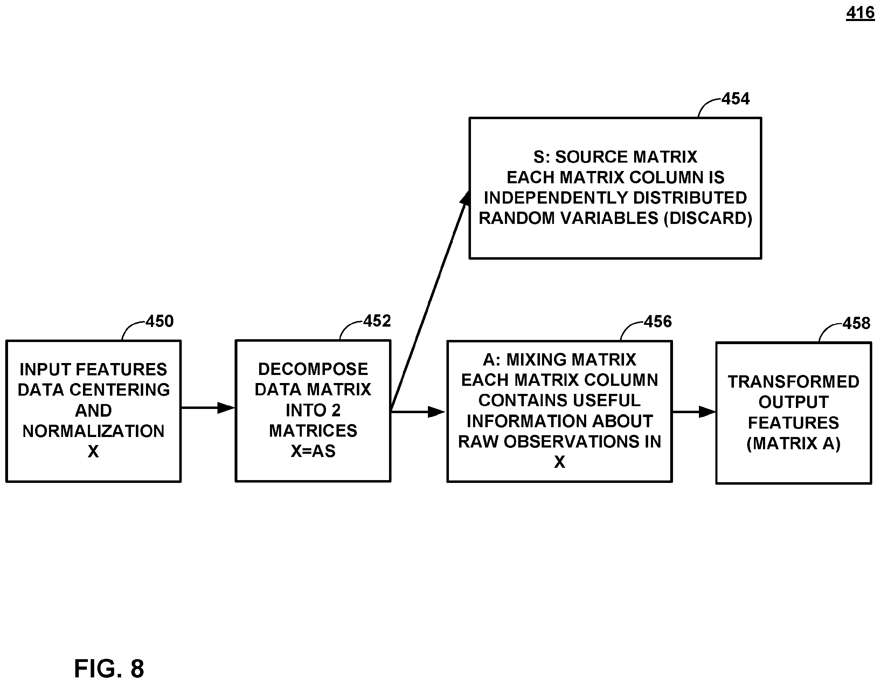

Similarly, controller device 150, for example, electrical parameters unit 152, may apply a reconstruction independent component analysis (RICA) algorithm (416) to generate RICA features 418 for identifying clusters. An example process of applying a RICA algorithm is shown in FIG. 8. RICA may refer to an unsupervised feature learning algorithm. In some examples, the RICA algorithm may use a ranked list of features. For instance, controller device 150 may determine a correlation of in vitro features to in vivo features and select a weight value for in vitro features based on the correlation of the in vitro features to the in vivo features. In some instances, controller device 150 may set a weight value for an in vitro feature to be higher as the correlation of the in vitro feature to the in vivo features increases and may set the weight value for an in vitro feature to be lower as the correlation of the in vitro feature to the in vivo features decreases. An example of RICA may be found at ufldl. ord. edu/tutorial/unsupervised/RICA/.

Controller device 150, for example, cluster identification unit 154, may identify a cluster for the glucose sensor (410). For example, controller device 150 may apply a machine learning algorithm, such as, for example, a least square of averages (kmeans), a hierarchical clustering, or spectral clustering to group similar sensors together.

Controller device 150 may validate a sensor glucose interim model 420. For example, controller device 150 may link an unsupervised learning output (e.g., cluster memberships) with the sensor glucose interim model 420 to validate the clustering performance and quantify feature importance. For example, controller device 150 may receive, for each cluster, one or more of a BG value, Isig, Vcounter, or EIS for each glucose sensor in a cluster. In this example, controller device 150 may determine, for each cluster, whether glucose sensors within the cluster satisfy a quality threshold. For example, controller device 150 may compare the BG value (e.g., from a finger stick) to an estimated value generated using Isig to generate a difference of BG value. In response to the difference of BG value for particular glucose sensor is greater than a threshold, controller device 150 may determine that the particular glucose sensor does not satisfy the quality threshold. In response, however, to the difference of BG value for particular glucose sensor is less than the threshold, controller device 150 may determine that the particular glucose sensor satisfies the quality threshold. While the above examples use BG, other values may be used by controller device 150 for validation, for example, controller device 150 may use MARD (e.g., a median MARD, or (interquartile range (IQR)MARD).

FIG. 7 is a conceptual diagram illustrating an example process of applying a PCA algorithm in accordance with one or more examples described in this disclosure. In the example of FIG. 7, controller device 150, for example, electrical parameters unit 152, may apply data centering and normalization (430). In this example, controller device 150, for example, electrical parameters unit 152, may compute a covariance matrix (432). For example, the covariance matrix is computed as:

$$Matrix(Covariance) = \begin{bmatrix} Var[X_1] & Cov[X_1, X_2] \\ Cov[X_2, X_1] & Var[X_2] \end{bmatrix}$$

EQUATION 1

Where X1 and X2 are examples of two features in the data sets X.

Controller device 150, for example, electrical parameters unit 152, may compute eigne values and eigen vectors (434). For example, $\lambda$ is an eigenvalue for the covariance matrix C if it is a solution of the characteristic equation:

$$\det(\lambda I - C) = 0$$

EQUATION 2

Where, I is the identity matrix of the same dimension as C which is a required condition for the matrix subtraction as well in this case and 'det' is the determinant of the matrix.

For each eigenvalue $\lambda$, a corresponding eigen-vector v, can be found by solving:

$$(\lambda I - C)v = 0$$

EQUATION 3

In this example, controller device 150, for example, electrical parameters unit 152, may arrange the eigen values and eigen vectors by principal components (436). For example, the eigenvector corresponding to the highest to lowest eigenvalue is the first to last principal component of the dataset. The principle components may be the eigen vectors sorted by the eigen value.

FIG. 8 is a conceptual diagram illustrating an example process of applying a RICA algorithm in accordance with one or more examples described in this disclosure. The RICA function, when executed by controller device 150, may create a linear transformation of input features to output features. The linear transformation of input features to output features may be based on optimizing a nonlinear objective function that roughly balances statistical independence of the output features versus the ability to reconstruct the input data using the output features.

Controller device 150, for example, electrical parameters unit 152, may determine input features and apply data centering and normalization to 'X' (450). In this example, controller device 150, for example, electrical parameters unit 152, may decompose the data matrix ('X') into 2 matrices ("X=AS") (452). Controller device 150, for example, electrical parameters unit 152, may determine a source matrix ('S'), where each matrix column is independently distributed random variables (discard) (454). In this example, controller device 150, for example, electrical parameters unit 152, may determine a mixing matrix ('A'), where each matrix column contains useful information about raw observations in 'X' (456). Controller device 150, for example, electrical parameters unit 152, may determine transformed output features ("MATRIX A") (458).

For example, Let $\Sigma$ = Cov(X) and suppose X = AS, and B = A^-1. In this example, B = W$\Sigma$^- 1/2 for some nonsingular W. Accordingly, S = BX = W$\Sigma$^- 1/2 X with Cov(S) = I and W orthonormal. Operationally controller device 150 may sphere the data X~ = $\Sigma$^- 1/2 X, and then seek an orthogonal matrix W so that the components S = WX~ are independent.

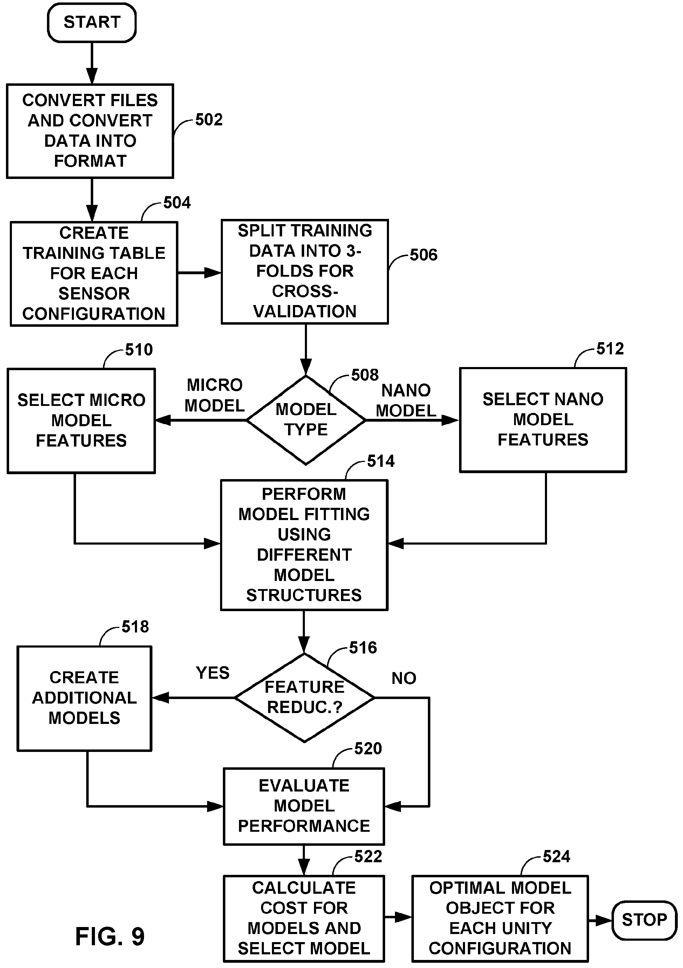

FIG. 9 is a conceptual diagram illustrating an example of model validation. The example of FIG. 9 may be an example of a process performed by SG interim model 420 of FIG. 6. In the example of FIG. 9, controller device 150 may convert files and convert data into a format (502). For example, controller device 150 may unify files in SVN (G2, G4, IRM10, IRM50, and UVGox) and convert unity data into a GS3-like format. Controller device 150 may create a training table for each sensor configuration (504). For instance, controller device 150 may create a training table for each sensor configuration with up to day 10 and applied outlier removal using 4.5 sigma techniques. Controller device 150 may split the training data into 3-folds for cross-validation (506). For instance, controller device 150 may split the training data into a first data set, a second data set, and a third data set. Controller device 150 may select a micro model ("micro model" of 508) and select micro model features (510). For example, controller device 150 may select one or more of blood glucose (BG), a time from of sensor relative to instruction time (e.g., age), electrical current (Isig), voltage (Vcounter), a first previous electrical current (IsigPrevI), a second previous electrical current (IsigPrev2), a third previous electrical current (1sigPrev3), a fourth previous electrical current (1sigPrev4), and one or more impedance values. Examples of impedance values may include a magnitude value and/or phase value.

Similarly, controller device 150 may select a nano model ("nano model" of 508) and select nano model features (512). For example, controller device 150 may select one or more of blood glucose (BG), an age of the glucose sensor (age), electrical current (Isig), voltage (Vcounter), a first previous electrical current (IsigPrevI), a second previous electrical current (IsigPrev2), a third previous electrical current (1sigPrev3), or a fourth previous electrical current (1sigPrev4).

Controller device 150 may perform model fitting using different model structures (514). For example, controller device 150 may perform model fitting using one or more of a linear model structure, a quadratic model structure, a linear and interactions model structure, and a linear, interactions, and quadratic model structure on the three different training data sets (e.g., the first data set, the second data set, and the third data set).

Controller device 150 may determine whether to perform a feature reduction process (516). In response to determining to perform the feature reduction process ("Yes" of step 516), controller device 150 may create additional models (518). For example, controller device 150 may create four additional models using stepwise regression to down select relevant features. In some examples, the additional models may have the same structure as above as the highest complexity but may result in simpler versions. In response, however, to determining to not perform the feature reduction process ("No" of step 516), controller device 150 skip creating additional models.

Controller device 150 may evaluate the model performance (520). For example, controller device 150 may evaluate one or more of a mean absolute relative difference (MARD), bias, or a standard deviation of MARD (e.g., std(MARD)) on the 3 different hold-out sets. Controller device 150 may calculate costs for models and select a model (522). For example, controller device 150 may calculate J=0.5 MARD + 0.5 std(MARD) and select a model that minimizing the cost 'J'. Controller device 150 may optimize the model object for each unity configuration (524).

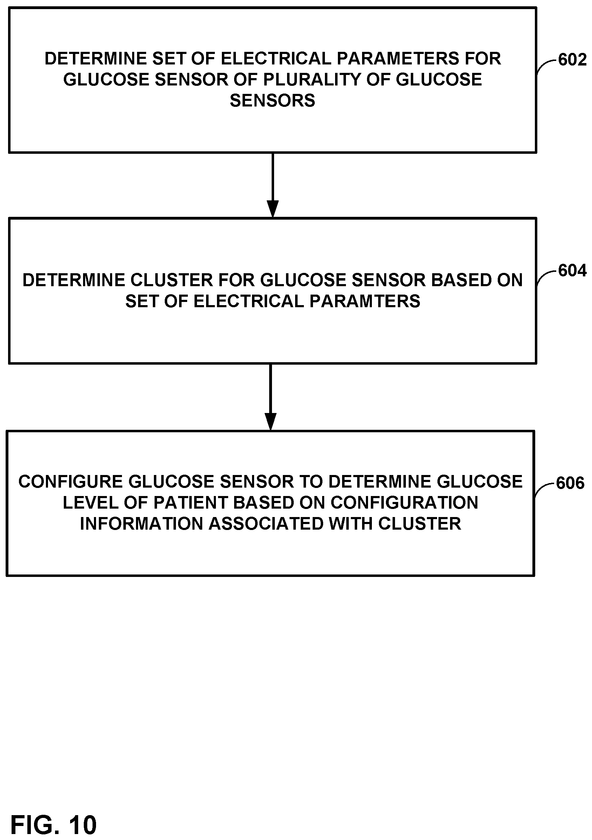

FIG. 10 is a flowchart illustrating an example technique of the disclosure. In the example of FIG. 10, controller device 150 may determine a set of electrical parameters for a glucose sensor (e.g., a sensor of monitoring device 100) (602). In some examples, the set of electrical parameters may comprise a voltage at the glucose sensor, an electrical current for the glucose sensor, or an impedance for the glucose sensor.

Controller device 150 may determine a cluster for the glucose sensor from a plurality of clusters based on the set of electrical parameters (604). Each cluster of the plurality of clusters identifies respective configuration information. For example, controller device 150 may apply a machine learning algorithm, where the machine learning algorithm has been trained using in vitro features and in vivo features for a training set of glucose sensors. For instance, controller device 150 may apply a RICA algorithm to the set of electrical parameters, where the RICA algorithm has been trained using the in vitro features and the in vivo features for the training set of glucose sensors. In some instances, controller device 150 may apply a PCA algorithm to the set of electrical parameters, where the PCA algorithm has been trained using the in vitro features and the in vivo features for the training set of glucose sensors. Controller device 150 may apply both RICA and PCA to the set of electrical parameters, where the RICA algorithm and the PCA algorithm have been trained using the in vitro features and the in vivo features for the training set of glucose sensors. Controller device 150 may train the machine learning algorithm (e.g., a RICA algorithm and/or a PCA algorithm) using in the vitro features and the in vivo features for the training set of sensor devices.

In some examples, controller device 150 may determine a cluster for each glucose sensor of the training set of glucose sensors based on in vitro features and in vivo features for the training set of sensor devices and apply a validation model (e.g., SG interim model 420) to verify the cluster for each glucose sensor of the training set of glucose sensors. In this example, controller device 150 may determine the cluster for the glucose sensor based on the in vitro features for the training set of sensor devices in each respective cluster.

Controller device 150 may configure the glucose sensor to determine a glucose level of a patient based on configuration information associated with the cluster (606). In some examples, the configuration information comprises a correction factor determined based on a subset of sensor devices of the training set of glucose sensors that are assigned to the cluster. Controller device 150 may output an indication of the cluster. In this example, the glucose sensor may be configured to determine the configuration information based on the cluster and to configure the glucose sensor with the configuration information.

In some examples, controller device 150 may determine a second set of electrical parameters for a second glucose sensor of the plurality of glucose sensors. In this example, controller device 150 may determine a second cluster for the second glucose sensor based on the second set of electrical parameters. For example, controller device 150 may apply a machine learning algorithm to determine the second cluster. Controller device 150 may determine that the second glucose sensor does not satisfy a quality metric in response to determining that the second glucose sensor is associated with the second cluster and that the second cluster is associated with quality value that does not satisfy the quality metric. In this way, controller device 150 may prune glucose sensors that do not satisfy a quality metric, which may improve a yield of glucose sensors.

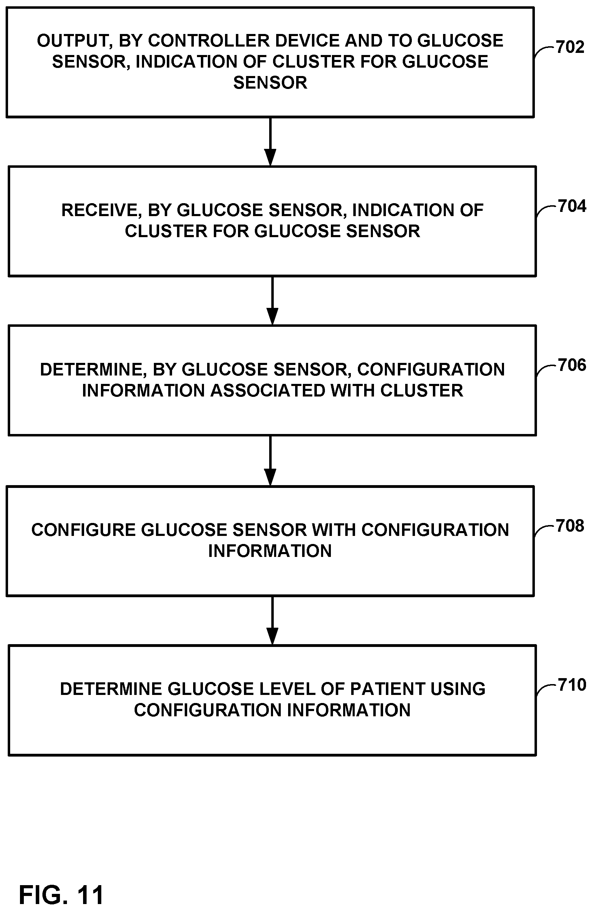

FIG. 11 is a flowchart illustrating another example technique of the disclosure. In the example of FIG. 11, controller device 150 may output an indication of a cluster for sensor electronics device 130 (702). Sensor electronics device 130 may receive the indication of the cluster (704) and determine configuration information associated with the cluster (706). For example, sensor electronics device 130 may determine configuration information 142 that is associated (e.g., using a look-up table) with the cluster. In this example, sensor electronics device 130 may configure sensor electronics device 130 with the configuration information (708) and determines a glucose level of patient 112 using the configuration information (710).

The techniques described in this disclosure may be implemented, at least in part, in hardware, software, firmware or any combination thereof. For example, various aspects of the described techniques may be implemented within one or more processors, including one or more microprocessors, digital signal processors (DSPs), application specific integrated circuits (ASICs), field programmable gate arrays (FPGAs), or any other equivalent integrated or discrete logic circuitry, as well as any combinations of such components. The term "processor" or "processing circuitry" may generally refer to any of the foregoing logic circuitry, alone or in combination with other logic circuitry, or any other equivalent circuitry. A control unit comprising hardware may also perform one or more of the techniques of this disclosure.

Such hardware, software, and firmware may be implemented within the same device or within separate devices to support the various operations and functions described in this disclosure. In addition, any of the described units, modules or components may be implemented together or separately as discrete but interoperable logic devices. Depiction of different features as modules or units is intended to highlight different functional aspects and does not necessarily imply that such modules or units must be realized by separate hardware or software components. Rather, functionality associated with one or more modules or units may be performed by separate hardware or software components, or integrated within common or separate hardware or software components.

The techniques described in this disclosure may also be embodied or encoded in a computer-readable medium, such as a computer-readable storage medium, containing instructions. Instructions embedded or encoded in a computer-readable medium may cause a programmable processor, or other processor, to perform the method, e.g., when the instructions are executed. Computer-readable media may include non-transitory computer-readable storage media and transient communication media. Computer readable storage media, which is tangible and non-transitory, may include random access memory (RAM), read only memory (ROM), programmable read only memory (PROM), erasable programmable read only memory (EPROM), electronically erasable programmable read only memory (EEPROM), flash memory, a hard disk, a CD-ROM, a floppy disk, a cassette, magnetic media, optical media, or other computer-readable storage media. It should be understood that the term "computer-readable storage media" refers to physical storage media, and not signals, carrier waves, or other transient media.

Various examples have been described. These and other examples are within the scope of the following claims.

## Claims

1. A method for calibrating a glucose sensor (12, 130), the method comprising:

   determining, by one or more processors, a set of electrical parameters for the glucose sensor, wherein the electrical parameters comprise a voltage at the glucose sensor, an electrical current for the glucose sensor, or an impedance for the glucose sensor;
   determining, by the one or more processors, a cluster for the glucose sensor (12, 130) from a plurality of clusters based on the set of electrical parameters, wherein each cluster of the plurality of clusters identifies respective configuration information; and
   configuring, by the one or more processors, the glucose sensor to determine a glucose level of a patient based on configuration information (142) identified by the determined cluster.

2. The method of claim 1, wherein determining the cluster comprises applying a machine learning algorithm, wherein the machine learning algorithm has been trained using in vitro features and in vivo features for a training set of glucose sensors.

3. The method of claim 2, wherein applying the machine learning algorithm comprises applying a reconstruction independent component analysis (RICA) algorithm to the set of electrical parameters, wherein the RICA algorithm has been trained using the in vitro features and the in vivo features for the training set of glucose sensors.

4. The method of claim 2, wherein applying the machine learning algorithm comprises applying a principal components analysis (PCA) algorithm to the set of electrical parameters, wherein the PCA algorithm has been trained using the in vitro features and the in vivo features for the training set of glucose sensors.

5. The method of claim 2, wherein applying the machine learning algorithm comprises applying both reconstruction independent component analysis (RICA) and principal components analysis (PCA) to the set of electrical parameters, wherein the RICA algorithm and the PCA algorithm have been trained using the in vitro features and the in vivo features for the training set of glucose sensors.

6. The method of any of claims 2-5, further comprising training, by the one or more processors, the machine learning algorithm using in the vitro features and the in vivo features for the training set of sensor devices.

7. The method of any of claims 2-6, wherein the configuration information comprises a correction factor determined based on a subset of sensor devices of the training set of glucose sensors that are assigned to the cluster.

8. The method of claim 1, wherein the configuration information comprises a correction factor.

9. The method of any preceding claim, wherein the glucose sensor is a first glucose sensor, wherein the cluster is a first cluster, and the set of electrical parameters is a first set of electrical parameters, the method further comprising:

   determining, by the one or more processors, a second set of electrical parameters for a second glucose sensor of the plurality of glucose sensors;
   determining, by the one or more processors, a second cluster of the plurality of clusters for the second glucose sensor based on the second set of electrical parameters;
   determining, by the one or more processors, that the second glucose sensor does not satisfy a quality metric in response to determining that the second glucose sensor is associated with the second cluster and that the second cluster is associated with quality value that does not satisfy the quality metric; and
   outputting, by the one or more processors, an indication that the second glucose sensor does not satisfy the quality metric.

10. The method of any preceding claim, wherein configuring the glucose sensor (12, 130) comprises outputting an indication of the cluster.

11. A device for calibrating a glucose sensor (12, 130), the device comprising:

   a memory; and
   one or more processors implemented in circuitry and in communication with the memory, the one or more processors configured to perform the method of any preceding claim.

12. A computer-readable storage medium having stored thereon instructions that, when executed, configure a processor to perform the method of any of claims 1-10.

13. A computer program comprising instructions which, when the program is executed by a processor, cause the processor to carry out the method of any of claims 1-10.


**Patentansprüche**

1. Verfahren zum Kalibrieren eines Glukosesensors (12, 130), wobei das Verfahren umfasst: Bestimmen, durch einen oder mehrere Prozessoren, eines Satzes von elektrischen Parametern für den Glukosesensor, wobei die elektrischen Parameter eine Spannung an dem Glukosesensor, einen elektrischen Strom für den Glukosesensor oder eine Impedanz für den Glukosesensor umfassen;

   Bestimmen, durch den einen oder die mehreren Prozessoren, eines Clusters für den Glukosesensor (12, 130) aus einer Vielzahl von Clustern basierend auf dem Satz von elektrischen Parametern, wobei jeder Cluster aus der Vielzahl von Clustern jeweilige Konfigurationsinformationen identifiziert; und
   Konfigurieren, durch den einen oder die mehreren Prozessoren, des Glukosesensors, um einen Glukosespiegels eines Patienten basierend auf Konfigurationsinformationen (142) zu bestimmen, die durch den bestimmten Cluster identifiziert werden.

**2.** Verfahren nach Anspruch 1, wobei das Bestimmen des Clusters das Anwenden eines Machine-Learning-Algorithmus umfasst, wobei der Machine-Learning-Algorithmus unter Verwendung von In-vitro-Merkmalen und In-vivo-Merkmalen für einen Trainingssatz von Glukosesensoren trainiert wurde.

**3.** Verfahren nach Anspruch 2, wobei das Anwenden des Machine-Learning-Algorithmus das Anwenden eines Algorithmus für rekonstruktionsunabhängige Komponentenanalyse (RICA) auf den Satz von elektrischen Parametern umfasst, wobei der RICA-Algorithmus unter Verwendung der In-vitro-Merkmale und der In-vivo-Merkmale für den Trainingssatz von Glukosesensoren trainiert wurde.

**4.** Verfahren nach Anspruch 2, wobei das Anwenden des Machine-Learning-Algorithmus das Anwenden eines Algorithmus für Hauptkomponentenanalyse (PCA) auf den Satz von elektrischen Parametern umfasst, wobei der PCA-Algorithmus unter Verwendung der In-vitro-Merkmale und der In-vivo-Merkmale für den Trainingssatz von Glukosesensoren trainiert wurde.

**5.** Verfahren nach Anspruch 2, wobei das Anwenden des Machine-Learning-Algorithmus das Anwenden sowohl einer rekonstruktionsunabhängigen Komponentenanalyse (RICA) als auch einer Hauptkomponentenanalyse (PCA) auf den Satz von elektrischen Parametern umfasst, wobei der RICA-Algorithmus und der PCA-Algorithmus unter Verwendung der In-vitro-Merkmale und der In-vivo-Merkmale für den Trainingssatz von Glukosesensoren trainiert wurden.

**6.** Verfahren nach einem der Ansprüche 2 bis 5, ferner umfassend das Trainieren, durch den einen oder die mehreren Prozessoren, des Machine-Learning-Algorithmus unter Verwendung der In-vitro-Merkmale und der In-vivo-Merkmale für den Trainingssatz von Sensorvorrichtungen.

**7.** Verfahren nach einem der Ansprüche 2 bis 6, wobei die Konfigurationsinformationen einen Korrekturfaktor umfassen, der basierend auf einem Teilsatz von Sensorvorrichtungen des Trainingssatzes von Glukosesensoren bestimmt wird, die dem Cluster zugeordnet sind.

**8.** Verfahren nach Anspruch 1, wobei die Konfigurationsinformationen einen Korrekturfaktor umfassen.

**9.** Verfahren nach einem der vorstehenden Ansprüche, wobei der Glukosesensor ein erster Glukosesensor ist, wobei der Cluster ein erster Cluster ist und der Satz von elektrischen Parametern ein erster Satz von elektrischen Parametern ist, wobei das Verfahren ferner umfasst:

Bestimmen, durch den einen oder die mehreren Prozessoren, eines zweiten Satzes von elektrischen Parametern für einen zweiten Glukosesensor aus der Vielzahl von Glukosesensoren;
Bestimmen, durch den einen oder die mehreren Prozessoren, eines zweiten Clusters aus der Vielzahl von Clustern für den zweiten Glukosesensor basierend auf dem zweiten Satz von elektrischen Parametern;
Bestimmen, durch den einen oder die mehreren Prozessoren, dass der zweite Glukosesensor eine Qualitätsmetrik nicht erfüllt, als Reaktion auf das Bestimmen, dass der zweite Glukosesensor dem zweiten Cluster zugeordnet ist und dass der zweite Cluster einem Qualitätswert zugeordnet ist, der die Qualitätsmetrik nicht erfüllt; und
Ausgeben, durch den einen oder die mehreren Prozessoren, einer Anzeige, dass der zweite Glukosesensor die Qualitätsmetrik nicht erfüllt.

**10.** Verfahren nach einem der vorstehenden Ansprüche, wobei das Konfigurieren des Glukosesensors (12, 130) das Ausgeben einer Anzeige des Clusters umfasst.

**11.** Vorrichtung zum Kalibrieren eines Glukosesensors (12, 130), wobei die Vorrichtung umfasst:

einen Speicher; und
einen oder mehrere Prozessoren, die in Schaltlogik implementiert sind und mit dem Speicher in Kommunikation stehen, wobei der eine oder die mehreren Prozessoren konfiguriert sind, um das Verfahren nach einem der vorstehenden Ansprüche durchzuführen.

**12.** Computerlesbares Speichermedium, das darauf gespeicherte Anweisungen aufweist, die, wenn sie ausgeführt werden, einen Prozessor konfigurieren, um das Verfahren nach einem der Ansprüche 1 bis 10 durchzuführen.

13. Computerprogramm, umfassend Anweisungen, die, wenn das Programm von einem Prozessor ausgeführt wird, den Prozessor dazu veranlassen, das Verfahren nach einem der Ansprüche 1 bis 10 durchzuführen.

**Revendications**

1. Procédé d'étalonnage d'un capteur de glucose (12, 130), le procédé comprenant :

   la détermination, par un ou plusieurs processeurs, d'un ensemble de paramètres électriques pour le capteur de glucose, dans lequel les paramètres électriques comprennent une tension au niveau du capteur de glucose, un courant électrique pour le capteur de glucose, ou une impédance pour le capteur de glucose ;
   la détermination, par le ou les processeurs, d'un regroupement pour le capteur de glucose (12, 130) d'une pluralité de grappes sur la base de l'ensemble de paramètres électriques, dans lequel chaque grappe de la pluralité de grappes identifie des informations de configuration respectives ; et
   la configuration, par le ou les processeurs, du capteur de glucose pour déterminer un niveau de glucose d'un patient sur la base d'informations de configuration (142) identifiées par le groupe déterminé.

2. Procédé selon la revendication 1, dans lequel la détermination du regroupement comprend l'application d'un algorithme d'apprentissage automatique, dans lequel l'algorithme d'apprentissage automatique a été entraîné à l'aide de caractéristiques in vitro et de caractéristiques in vivo pour un ensemble d'entraînement de capteurs de glucose.

3. Procédé selon la revendication 2, dans lequel l'application de l'algorithme d'apprentissage automatique comprend l'application d'un algorithme d'analyse de composantes indépendantes de la reconstruction (RICA) à l'ensemble de paramètres électriques, dans lequel l'algorithme RICA a été entraîné à l'aide des caractéristiques in vitro et des caractéristiques in vivo pour l'ensemble d'entraînement de capteurs de glucose.

4. Procédé selon la revendication 2, dans lequel l'application de l'algorithme d'apprentissage automatique comprend l'application d'un algorithme d'analyse en composantes principales (ACP) à l'ensemble de paramètres électriques, dans lequel l'algorithme ACP a été entraîné à l'aide des caractéristiques in vitro et des caractéristiques in vivo pour l'ensemble d'entraînement de capteurs de glucose.

5. Procédé selon la revendication 2, dans lequel l'application de l'algorithme d'apprentissage automatique comprend l'application à la fois d'une analyse en composantes indépendantes de la reconstruction (RICA) et d'une analyse en composantes principales (ACP) à l'ensemble de paramètres électriques, dans lequel l'algorithme RICA et l'algorithme PCA ont été entraînés à l'aide des caractéristiques in vitro et des caractéristiques in vivo pour l'ensemble d'entraînement de capteurs de glucose.

6. Procédé selon l'une quelconque des revendications 2 à 5, comprenant en outre l'entraînement, par le ou les processeurs, de l'algorithme d'apprentissage automatique à l'aide des caractéristiques in vitro et des caractéristiques in vivo pour l'ensemble d'entraînement de dispositifs de capteurs.

7. Procédé selon l'une quelconque des revendications 2 à 6, dans lequel les informations de configuration comprennent un facteur de correction déterminé en fonction d'un sous-ensemble de dispositifs de capteur de l'ensemble d'entraînement de capteurs de glucose qui sont affectés au regroupement.

8. Procédé selon la revendication 1, dans lequel les informations de configuration comprennent un facteur de correction.

9. Procédé selon l'une quelconque revendication précédente, dans lequel le capteur de glucose est un premier capteur de glucose, dans lequel le groupe est un premier groupe, et l'ensemble de paramètres électriques est un premier ensemble de paramètres électriques, le procédé comprenant en outre :

   la détermination, par le ou les processeurs, d'un second ensemble de paramètres électriques pour un second capteur de glucose de la pluralité de capteurs de glucose ;
   la détermination, par le ou les processeurs, d'un deuxième groupe de la pluralité de groupes pour le second capteur de glucose sur la base du second ensemble de paramètres électriques ;
   le fait de déterminer, par le ou les processeurs, que le second capteur de glucose ne satisfait pas à une métrique de qualité en réponse à la détermination que le second capteur de glucose est associé au second regroupement et que le second regroupement est associé à une valeur de qualité qui ne satisfait pas à la métrique de qualité ; et
la sortie, par le ou les processeurs, d'une indication que le second capteur de glucose ne satisfait pas à la
métrique de qualité.

**10.** Procédé selon l'une quelconque revendication précédente, dans lequel la configuration du capteur de glucose (12, 130) comprend l'émission d'une indication du regroupement.

**11.** Dispositif d'étalonnage d'un capteur de glucose (12, 130), le dispositif comprenant :

une mémoire ; et
un ou plusieurs processeurs mis en œuvre dans un système de circuit et en communication avec la mémoire, le ou les processeurs étant configurés pour effectuer le procédé selon l'une quelconque revendication précédente.

**12.** Support de stockage lisible par un ordinateur sur lequel sont stockées des instructions qui, lorsqu'elles sont exécutées, configurent un processeur pour effectuer le procédé selon l'une quelconque des revendications 1 à 10.

**13.** Programme informatique comprenant des instructions qui, lorsque le programme est exécuté par un processeur, amènent le processeur à réaliser le procédé selon l'une quelconque des revendications 1 à 10.

110

112

118

100

116

114

PATIENT
DEVICE
**124**

**FIG. 1**

FIG. 2

EP 4 111 965 B1

FIG. 3

EP 4 111 965 B1

111

130A

130B

130N

MEASUREMENT
PROCESSOR
132

DISPLAY AND
TRANSMISSION
UNIT
134

CONTROLLER
136

POWER SUPPLY
138

MEMORY
140

CONFIGURATION
INFORMATION
142

CONTROLLER DEVICE
150

ELECTRICAL
PARAMETERS
UNIT
152

CLUSTER
IDENTIFICATION
UNIT
154

CONFIGURATION
UNIT
156

FIG. 4

DOMAIN

<u>STEP 1:</u>
FEATURE
IDEATION

<u>STEP 2:</u>
FEATURE
SELECTION IN
DOMAIN

<u>STEP 3:</u>
INITIAL
FEATURE
SELECTION BY
IVIV
RELATIONSHIP

<u>IN VIVO</u>

302
IN VIVO
FEATURES

304
IDENTIFY
IMPORTANT
IN VIVO
FEATURES

320
CORRELATE
IN VITRO AND
IN VIVO
FEATURES

322
INITIAL IN
VITRO AND IN
VIVO
FEATURES

<u>IN VITRO</u>

312
IN VITRO
FEATURES

314
IDENTIFY
IMPORTANT
IN VITRO
FEATURES

**FIG. 5**

FIG. 6

EP 4 111 965 B1

┌─────────────────┐        ┌─────────────────┐        ┌─────────────────┐        ┌─────────────────┐
│      430        │        │      432        │        │      434        │        │      436        │
│                 │        │                 │        │                 │        │                 │
│ DATA CENTERING  │        │    COMPUTE      │        │    COMPUTE      │        │  ARRANGE BY     │
│      AND        │ ─────▶ │  COVARIANCE     │ ─────▶ │  EIGENVALUES    │ ─────▶ │  PRINCIPAL      │
│ NORMALIZATION   │        │    MATRIX       │        │      AND        │        │  COMPONENTS     │
│                 │        │                 │        │  EIGENVECTORS   │        │                 │
└─────────────────┘        └─────────────────┘        └─────────────────┘        └─────────────────┘

**FIG. 7**

EP 4 111 965 B1

416

454

S: SOURCE MATRIX
EACH MATRIX COLUMN IS
INDEPENDENTLY DISTRIBUTED
RANDOM VARIABLES (DISCARD)

450

INPUT FEATURES
DATA CENTERING
AND
NORMALIZATION
X

452

DECOMPOSE
DATA MATRIX
INTO 2
MATRICES
X=AS

456

A: MIXING MATRIX
EACH MATRIX COLUMN
CONTAINS USEFUL
INFORMATION ABOUT
RAW OBSERVATIONS IN
X

458

TRANSFORMED
OUTPUT
FEATURES
(MATRIX A)

FIG. 8

EP 4 111 965 B1

FIG. 9

DETERMINE SET OF ELECTRICAL PARAMETERS FOR GLUCOSE SENSOR OF PLURALITY OF GLUCOSE SENSORS ⟋602

DETERMINE CLUSTER FOR GLUCOSE SENSOR BASED ON SET OF ELECTRICAL PARAMTERS ⟋604

CONFIGURE GLUCOSE SENSOR TO DETERMINE GLUCOSE LEVEL OF PATIENT BASED ON CONFIGURATION INFORMATION ASSOCIATED WITH CLUSTER ⟋606

FIG. 10

OUTPUT, BY CONTROLLER DEVICE AND TO GLUCOSE SENSOR, INDICATION OF CLUSTER FOR GLUCOSE SENSOR ⟋702

RECEIVE, BY GLUCOSE SENSOR, INDICATION OF CLUSTER FOR GLUCOSE SENSOR ⟋704

DETERMINE, BY GLUCOSE SENSOR, CONFIGURATION INFORMATION ASSOCIATED WITH CLUSTER ⟋706

CONFIGURE GLUCOSE SENSOR WITH CONFIGURATION INFORMATION ⟋708

DETERMINE GLUCOSE LEVEL OF PATIENT USING CONFIGURATION INFORMATION ⟋710

## FIG. 11

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20120262298 A1
- US 20040223155 A1